# EUROPEAN PATENT APPLICATION

(11) **EP 4 775 254 A1**
(43) Date of publication of application: **15.07.2026**
(21) Application number: 24862836.4
(22) Date of filing: 04.09.2024
(51) Int. Cl.: A61M 37/00, A61K 9/70, A61K 31/245, A61K 47/32, A61K 47/34, A61P 23/02

(54) **ORAL LOCALIZED APPLICATION SYSTEM**

(30) Priority: 04.09.2023 JP 2023142766
(71) Applicant: Cosmed Pharmaceutical Co., Ltd., Kyoto-shi, Kyoto 601-8438 (JP)
(72) Inventor: QUAN, Ying-shu, Kyoto-shi, Kyoto 601-8438 (JP); TANAKA, Hiroshi, Kyoto-shi, Kyoto 601-8438 (JP); SAITO, Mio, Kyoto-shi, Kyoto 601-8438 (JP); HASEGAWA, Junya, Kyoto-shi, Kyoto 601-8438 (JP); KAMIYAMA, Fumio, Kyoto-shi, Kyoto 601-8438 (JP)
(74) Representative: Ter Meer Steinmeister & Partner
(86) International application number: PCT/JP2024/031752
(87) International publication number: WO 2025/053184

(57) **Abstract**

The present invention provides a means for shortening an onset time of local anesthetics, antihistamines, and the like on oral mucosa.

The invention relates to a microprojection patch comprising a substrate and microprojections standing on the substrate, the microprojection patch being made of a water-insoluble thermoplastic polymer material and being suitable for application to oral mucosa, and also relates to a localized application system for shortening an onset time of a drug on oral mucosa, the system comprising the microprojection patch, a topical drug (such as a gel or ointment containing a drug), and an adhesive tape for backing.

## Description

### [Technical Field]

The present invention relates to a patch having microprojections for localized application to the oral mucosa. The present invention further relates to a localized application system using the patch.

### [Background Art]

Dental local anesthetics have been widely used similarly to local anesthetics for skin. As drugs, (gel) ointments containing lidocaine and ethyl aminobenzoate are generally used, and their onset time is 10 to 20 minutes, which is shorter than the onset time on skin. However, an onset of action in a shorter time has been demanded by both physicians and patients.

The present inventors developed a microneedle array containing a local anesthetic for oral application and succeeded in achieving an anesthetic effect in a short time (Patent Document 1). Patent Documents 1 and 2 disclose inventions of a microneedle formulation for skin in which a local anesthetic is dissolved in microneedles, or a formulation in which a local anesthetic is applied to microneedles. From the viewpoint of physicians, there has been a demand for a new administration method which enables physicians to administer various familiar local administration formulations composed of (gel) ointments and liquid preparations having a track record of use, and to develop an effect in a shorter time by appropriately preparing and applying the formulations at medical sites.

### [Prior Art Documents]

### [Patent Documents]

[Patent Document 1] Japanese Unexamined Patent Application Publication No. 2019-084352
[Patent Document 2] Japanese Unexamined Patent Application Publication No. 2017-061447

### [Summary of Invention]

### [Problems to be Solved by the Invention]

The present invention has been made to solve problems relating to localized application preparations, and an object thereof is to provide means for shortening the onset time upon intraoral administration of locally administrable preparations such as ointments, creams, liquid preparations, gels and the like for local administration for the purpose of local anesthesia, antihistaminic effect, anti-inflammatory effect, antibacterial effect, pain relief, antipruritic effect, and the like.

### [Means for Solving the Problems]

In the present invention, administration sites of the localized application preparations are the oral cavity and gingiva. Since the outermost layer of the oral mucosa or gingiva is keratinized stratified squamous epithelium and is mucosa, it is thinner than the stratum corneum of normal skin, and thus percutaneous absorption of a local anesthetic is higher than that of normal skin, while it is delicate in that it is prone to feel pain and to bleed. On the other hand, the oral mucosa or gingiva has less planarity and more irregularities than normal skin, and thus it is difficult to stably retain a preparation on an affected area. Further, since it is in the oral cavity, it is constantly exposed to moisture of saliva, and thus protection against peeling of a patch-type preparation and outflow of an applied preparation is required. Since there are differences in conditions as described above, technical problems in localized application preparations to the oral mucosa include problems common to preparations applied to normal skin, but also include many technical problems completely different therefrom.

The present inventors developed a microprojection patch in order to solve the above problems. In order to shorten an onset time of local anesthetics, antihistamines, anti-inflammatory agents, antibacterial agents, pain relieving agents, antipruritic agents, and the like on the oral mucosa, when applying (gel) ointments or liquid preparations containing these drugs to an affected area in the oral cavity, the microprojection patch of the present invention is overlaid on the applied site. Upon application of a local anesthetic composed of a solution to skin, if the viscosity of the solution is too low, even when applied to the oral cavity, the solution may immediately flow away on the mucosal surface and the anesthetic may not remain only at the intended local site. In order to avoid this, it is desirable that the applied preparation be applied as a high-viscosity preparation so as to appropriately remain on the applied surface.

As the microprojection patch, a microneedle patch is known. Materials constituting microneedles include silica, metals, plastics, biodegradable substances, and the like, and conventional microneedles are characterized by having hardness enabling skin puncture. In the present invention, the microprojections may have hardness enabling puncture even when applied to the oral mucosa, but preferably have hardness not enabling puncture. In the case of mucosal puncture, a drug effectively permeates into the oral mucosa through micropores formed in the oral mucosa. In a soft microneedle that cannot puncture the oral mucosa, the microprojections press the oral mucosa to locally stretch and thin the oral mucosa, thereby improving mucosal absorption of a drug to be administered simultaneously. If the microneedle is made of a material having low hardness and flexibility, even when the microneedle is punctured into the oral mucosa, the puncture depth is only within several µm from the surface, and thus pain is not felt and bleeding does not occur.

As a material constituting the microprojection patch of the present invention, a water-insoluble polymer is used. Even if a microprojection patch is formed using a water-soluble polymer, for example, hyaluronic acid or carboxymethyl cellulose, when applied to moist oral mucosa, a tip of the microprojections and then most of the needle are softened and dissolved by moisture, and thus the oral mucosa cannot be deformed or punctured. Therefore, the onset time of a drug, which is the purpose of the present invention, cannot be shortened.

The microprojection patch may be backed with an adhesive sheet. The adhesive sheet strongly adheres to the oral mucosa in the presence of saliva, thereby enabling puncture of the microprojections or compression of the skin.

The present invention is as follows.
[1] A microprojection patch comprising a substrate and microprojections standing on the substrate, the microprojection patch being made of a water-insoluble thermoplastic polymer material and being used for application to oral mucosa.
[2] The microprojection patch according to [1], wherein a tip diameter of the microprojections is from 10 µm to 300 µm, a length of the microprojections is from 50 µm to 1500 µm, and a thickness of the substrate is from 20 µm to 1000 µm.
[3] The microprojection patch according to [1] or [2], wherein a density of the microprojections is from 10 projections/cm² to 1000 projections/cm².
[4] The microprojection patch according to any one of [1] to [3 ] , wherein the water-insoluble thermoplastic polymer material is selected from the group consisting of polyolefins, soft silicone, and polyurethane.
[5] The microprojection patch according to any one of [1] to [3 ] , wherein the water-insoluble thermoplastic polymer material is selected from the group consisting of polyvinyl chloride, polyester, polyacetal, polylactic acid, lactic acid-glycolic acid copolymer (PLGA), and polyglycolic acid.
[6] The microprojection patch according to any one of [1] to [5 ] , wherein the substrate has flexibility, and the water-insoluble thermoplastic polymer material has an elastic modulus of 5.0 × 10⁴ kgf/cm² or less.
[7] An adhesive-tape-backed microprojection patch comprising the microprojection patch according to any one of [1] to [6] and an adhesive tape, wherein the microprojection patch is backed with the adhesive tape.
[8] The adhesive-tape-backed microprojection patch according to [7] , wherein an adhesive constituting the adhesive tape contains a plasticizer.
[9] The adhesive-tape-backed microprojection patch according to [7] or [8] , wherein an adhesive constituting the adhesive tape contains a hydrophilic substance.
[10] The adhesive-tape-backed microprojection patch according to [9] , wherein the hydrophilic substance is at least one selected from the group consisting of polyacrylic acid, hyaluronic acid and sodium salts thereof, carboxymethyl cellulose and sodium salts thereof, polyvinyl alcohol, polyvinyl pyrrolidone, dextrin, sodium chondroitin sulfate, collagen, and crosslinked products thereof.
[11] The adhesive-tape-backed microprojection patch according to any one of [7] to [10] , wherein a base material for the adhesive constituting the adhesive tape is a foam.
[12] The adhesive-tape-backed microprojection patch according to any one of [7] to [10] , wherein a base material for the adhesive constituting the adhesive tape is a nonwoven fabric.
[13] A localized application system for shortening a drug onset time on oral mucosa, comprising the microprojection patch according to any one of [1] to [6] and a topical drug.
[14] A localized application system for shortening a drug onset time on oral mucosa, comprising the adhesive-tape-backed microprojection patch according to any one of [7] to [10] and a topical drug.
[15] A localized application system for shortening a drug onset time on oral mucosa, comprising the microprojection patch according to any one of [1] to [6] and a coating mixture.
[16] A localized application system for shortening a drug onset time on oral mucosa, comprising the adhesive-tape-backed microprojection patch according to any one of [7] to [10] and a coating mixture.
[17] The localized application system according to [15] or [16 ] , wherein a viscosity of the coating mixture is from 15 mPa·s to 300,000 mPa·s.
[18] The localized application system according to [13] or [14 ] , wherein a drug contained in the topical drug is a local anesthetic or an antihistamine, and the system is intended to achieve pain relief or itch relief in a short time.
[19] The localized application system according to [16] or [17 ] , wherein a drug contained in the coating mixture is a local anesthetic or an antihistamine, and the system is intended to achieve pain relief or itch relief in a short time.
[20] The localized application system according to [18] or [19 ] , wherein the local anesthetic is lidocaine or ethyl aminobenzoate.
[21] The localized application system according to [18] or [19 ] , wherein the antihistamine is selected from the group consisting of diphenhydramine and salts thereof, and chlorpheniramine and salts thereof.

### [Advantageous Effects of Invention]

The localized application system of the present invention can shorten an onset time of a topical drug such as a local anesthetic, an antihistamine, an anti-inflammatory agent, an antibacterial agent, a pain relieving agent, an antipruritic agent, and the like on oral mucosa.

### [Brief Description of Drawings]

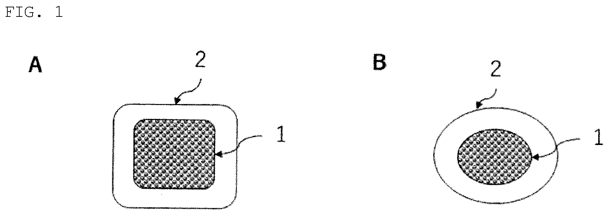

[Fig. 1] Fig. 1 is a schematic view showing an adhesive-tape-backed microprojection patch produced in Example 1.

### [Description of Embodiments]

The microprojection patch of the present invention for application to oral mucosa is water-insoluble. When the microprojection patch is water-insoluble, the microprojections do not dissolve or swell even when the microprojections are in contact with oral mucosa for several minutes to several tens of minutes. Therefore, water-insolubility is a necessary requirement for the material of the present invention. In a water-soluble microprojection, when applied to mucosa, the microprojections are likely to swell or dissolve due to moisture on the surface, and thus a drug may diffuse toward the mucosa side and, at the same time, reverse diffusion toward the microprojection portion may also occur.

A material of the microprojection patch of the present invention is a water-insoluble thermoplastic polymer material. Specifically, a microprojection patch made of polyolefins such as polyethylene (0.3-1.2), polypropylene (1.1-1.5), ethylene propylene rubber (0.9-1.3), soft silicone, polyurethane, and the like is suitable. The numbers in parentheses represent values of tensile elastic modulus (unit: 10⁴ kgf/cm²).

These resins are relatively soft thermoplastic polymer materials and are characterized in that they are less likely to puncture mucosa even when administered to the oral cavity. These thermoplastic polymer materials are characterized by having an elastic modulus of 1.5 × 10⁴ kgf/cm² or less.

Polyvinyl chloride, polycarbonate, polyester, 6-nylon, polyacetal, PS (polystyrene), PPS (polyphenylene sulfide), PAI (polyamideimide), PEEK (polyether ether ketone), PSU (polysulfone), polylactic acid, lactic acid-glycolic acid copolymer (PLGA), polyglycolic acid, and the like are hard, and microprojections produced from these materials are likely to puncture skin but can be used for the present purpose. The water-insoluble thermoplastic polymer material is also preferably selected from the group consisting of polyvinyl chloride, polyester, polyacetal, polylactic acid, lactic acid-glycolic acid copolymer (PLGA), and polyglycolic acid. These thermoplastic resins have an elastic modulus exceeding 1.5 × 10⁴ kgf/cm².

In particular, polyethylene and polypropylene have high biosafety and flexibility, and are more preferable as raw material resins for microneedles that do not puncture the oral cavity.

The microprojection patch includes a substrate and microprojections standing on the substrate. More specifically, in the microprojection patch, microprojections stand on a first surface of the substrate. In the microprojection patch, it is preferable that the substrate and the microprojections are integrally molded. That is, in the microprojection patch, it is preferable that the substrate and the microprojections are an integrally molded product made of a water-insoluble thermoplastic polymer.

A thickness of the substrate is from 20 µm to 1000 µm, preferably from 20 µm to 500 µm, and more preferably from 50 µm to 300 µm. When the thickness of the substrate is 20 µm or more, the microprojection patch is easy to handle. When the thickness of the substrate is 1000 µm or less, the microprojection patch can be easily brought into close contact with an oral mucosa portion having large irregularities. Further, when the thickness of the substrate is 20 µm or more and 500 µm or less, it is more preferable because the sheet is likely to fit irregularities in the oral cavity due to flexibility of the sheet.

A length of the microprojections is preferably from 50 µm to 1500 µm, and more preferably from 100 µm to 1000 µm. Further, in order to prevent deep puncture into oral mucosa, the length of the microprojections is more preferably 500 µm or less. When the length of the microprojections is 50 µm or more, the length is sufficient as a projection height for achieving a surface layer thinning effect and puncturing mucosa as needed. When the length of the microprojections is 1500 µm or less, bending of the microprojections can be prevented.

A shape of the microprojections is preferably conical or truncated conical. In the case of projections having such a shape, a tip diameter of the microprojections is preferably 10 µm or more and 300 µm or less, and more preferably from 30 µm to 200 µm.

A density of the microprojections is from 1 projection/cm² to 3000 projections/cm². The density is preferably from 10 projections/cm² to 1000 projections/cm², and more preferably from 50 projections/cm² to 800 projections/cm². When the density of the microprojections is 1 projection/cm² or more, effects of the present invention can be exhibited. When the density of the microprojections is 3000 projections/cm² or less, it is easy to sufficiently cope with irregularities of mucosa. When a normal coating gel or cream is placed on a tip portion of the microprojections and attached to mucosa, the density of the microprojections is preferably 50 projections/cm² or more. When the number of microprojections is small, in consideration of viscosity of a normal coating preparation, the preparation accumulates at a bottom surface rather than at the tip portion of the microprojections, thereby decreasing a contact area with mucosa and also decreasing permeability of the drug.

When a surface of oral mucosa is pressed by the microprojections, the oral mucosa is stretched in a concave manner and becomes thinner, whereby permeation of an applied drug into the oral cavity is remarkably improved. When the microprojections are hard and have an elastic modulus exceeding 1.5 × 10⁴ kgf/cm², the microprojections puncture the oral mucosa, and the drug highly permeates through the punctured holes. With respect to the substrate, when used on oral mucosa, a hard substrate can be used for a small-area treatment, whereas for a large-area treatment, a substrate made of a polymer material having flexibility and an elastic modulus of 5.0 × 10⁴ kgf/cm² or less is easier to attach. The projection portion may be made of a material having a different hardness as needed.

An area of the microprojection patch corresponds to an area of the substrate and is not particularly limited; however, for intraoral application, the area is preferably from 0.5 to 5 cm².

In the microprojection patch having such a configuration, an applied drug can be reliably retained at a target site, and drug administration efficiency can be remarkably improved. In the case of a local anesthetic, an onset time of an anesthetic effect can be shortened. In the case of pain relieving agents and antipruritic agents, shortening of a time required for pain relief and itch relief can be achieved.

For applying the microprojection patch to skin, the microprojection patch may be used while being backed with an adhesive tape around a periphery thereof in order to fix the microprojection patch in the oral cavity. Although the adhesive tape is not essential, fixation with an adhesive tape is preferable for stably fixing the microprojection patch to skin. That is, it is preferable to use an adhesive-tape-backed microprojection patch including the above-described microprojection patch and an adhesive tape, wherein the microprojection patch is backed with the adhesive tape. The phrase "the microprojection patch is backed with an adhesive tape" means that the adhesive tape is disposed on a second surface of a base material of the microprojection patch. As described above, a first surface of the base material of the microprojection patch is a surface on which the microprojections stand. The second surface of the base material is a surface opposite to the first surface of the base material.

The adhesive tape includes an adhesive and a base material (adhesive base material), and is formed by applying the adhesive onto the base material. The adhesive includes an adhesive component. Examples of the adhesive component of the adhesive include rubber-based adhesives, silicone-based adhesives, acrylic adhesives, urethane-based adhesives, and the like. Since the oral mucosa itself contains a large amount of moisture and the surrounding environment is extremely moist, the adhesive component used for the adhesive tape is preferably a hydrophilic adhesive component, and more preferably has strong adhesion to oral mucosa under wet surfaces and wet environments.

Examples of the hydrophilic adhesive component include a hydrophilic acrylic adhesive, HiPAS10 (trade name) adhesive (an acrylic adhesive using methyl methacrylate as a main monomer, CosMED Pharmaceutical), and HiPAS-PU (trade name) (a urethane-based adhesive, CosMED Pharmaceutical).

In the adhesive tape, a base material (adhesive base material) is preferably a base film. In this case, the adhesive may be applied directly onto the base film to form the adhesive tape. However, particularly for intraoral application, since the oral cavity is extremely hydrophilic and contains a large amount of water on its surface, the water may cover a surface of the adhesive and cause a loss of adhesiveness. In order to prevent this and to enable stable application to skin for a long period of time, it is effective to previously blend and mix a hydrophilic substance into the adhesive and apply the adhesive onto the base material.

In the present invention, the hydrophilic substance refers to a substance that dissolves or swells when added to water. That is, the adhesive preferably contains an adhesive component and a hydrophilic substance. Examples of the hydrophilic substance include low-molecular-weight hydrophilic substances such as glucose and glycerin; and hydrophilic polymer substances such as polyacrylic acid, hyaluronic acid (sodium salt), carboxymethyl cellulose (sodium salt), polyvinyl alcohol, polyvinyl pyrrolidone, dextrin, sodium chondroitin sulfate, collagen, and crosslinked products thereof. From the viewpoint of addition to a nonaqueous solution such as an acrylic adhesive, hydrophilic polymer substances are more preferable. The role of the hydrophilic substance is to absorb moisture in the oral cavity and maintain adhesiveness of the adhesive for a long period of time. The content of the hydrophilic substance is preferably from 3 to 80 mass% and more preferably from 5 to 70 mass%, based on 100 mass% of the adhesive. If the content of the hydrophilic substance is too small, the above effect is difficult to obtain, whereas if the content is too large, adhesiveness of the adhesive is lost and the hydrophilic substance tends to remain on skin. Use of an adhesive in which a large amount of a hydrophilic polymer is kneaded into a thermoplastic adhesive, such as a hydrocolloid, is also acceptable.

The adhesive may contain a plasticizer. As the plasticizer, commonly used plasticizers can be used, for example, isopropyl myristate, isopropyl palmitate, octyl myristate, octyldodecyl lactate, and the like.

From the viewpoint of application to bent portions such as skin and the oral cavity, a base material to which the adhesive is applied is preferably a flexible film, a foam, or a nonwoven fabric. In order to stabilize intraoral adhesion of the adhesive tape by absorption of moisture in the oral cavity, use of a foam or a nonwoven fabric is more preferable, and materials having higher water absorbency are more preferable. The foam can exert an effect of preventing flow of a preparation when used in an overlay manner with a cream preparation or a liquid preparation. Since the foam is thicker than a film, it is required that hardness of the foam is not excessively high so as not to cause discomfort in the oral cavity. When an elastic modulus of the foam is 20 kgf/cm² or less, no discomfort is caused in the oral cavity. Measurement of the elastic modulus can be performed based on a stress-strain curve obtained when a foam is punched into a dumbbell shape and pulled by a tensile tester. The foam may be either an open-cell foam or a closed-cell foam, as long as a side contacting the oral cavity is porous.

A thickness is preferably 300 µm or less and 5 µm or more for a film (or 100 µm or less and 5 µm or more), 10 mm or less and 0.1 mm or more for a foam, and 20 mm or less and 0.05 mm or more for a nonwoven fabric. More preferably, the thickness is 100 µm or less and 10 µm or more for a film, 6 mm or less and 0.5 mm or more for a foam, and 10 mm or less and 0.08 mm or more for a nonwoven fabric. Since a size of the adhesive/base material is for fixing the microprojection patch, the size is preferably from 1 to 10 mm beyond a periphery of the microprojection patch.

The localized application system of the present invention is a localized application system for shortening a drug onset time on oral mucosa, comprising the above-described microprojection patch and a topical drug. The microprojection patch may be backed with an adhesive tape. That is, the localized application system of the present invention may be a localized application system for shortening a drug onset time on oral mucosa, comprising the above-described adhesive-tape-backed microprojection patch and a topical drug.

In actual application, a topical drug (such as a gel or an ointment) is placed on a microprojection surface, spread to form a planar layer, applied to oral mucosa, and pressed from a back surface of the substrate to bring the microprojection patch into close contact with the oral mucosa. Alternatively, the topical drug may be directly applied to a predetermined site of the oral mucosa, and immediately thereafter pressed from above with the microprojection patch.

A shape of the microprojection patch may be square, rectangular, circular, elliptical, cylindrical, spherical, or the like. When the microprojection patch is backed with an adhesive tape and brought into close contact with the oral mucosa, adhesiveness is further improved. As a result, stretching of the oral mucosa and/or puncture thereof promotes permeation of the drug into the oral mucosa.

When the topical drug has an appropriately high viscosity and does not flow even when placed on the microprojection surface, the above-described administration method may be used. However, when the topical drug is a liquid preparation having low viscosity, it is preferable to perform the following pretreatment. Specifically, an appropriate amount of a hydrophilic substance (particularly a hydrophilic polymer substance) in powder form or a high-concentration aqueous solution of a hydrophilic substance is mixed with the liquid preparation, and the obtained coating mixture is placed on the microprojection surface.

In the present invention, a viscosity of the coating mixture is preferably from 15 mPa·s to 300,000 mPa·s, and more preferably from 20 mPa·s to 20,000 mPa·s.When the viscosity of the coating mixture is less than 15 mPa·s, fluidity of the coating mixture is too high, and the coating mixture flows away from an affected site, or it is difficult to quantitatively place the coating mixture on the microprojection surface. When the viscosity exceeds 300,000 mPa·s, fluidity of the coating mixture is too low, and it is difficult to uniformly place the coating mixture on the microprojection surface, and even when applied to the oral cavity, the coating mixture is unlikely to uniformly spread on an oral surface.

The viscosity of the coating mixture was measured at 25°C using a type E viscometer, and a value measured 2 minutes after the start of measurement was recorded.

For application to oral mucosa, the microprojection patch preferably has a shape that closely contacts and fits a dental bed. Specifically, when the microprojection patch is rectangular, a patch having a short side of 0.5 cm or more and a long side of 5 cm or less is preferable. When the microprojection patch is circular, a diameter thereof is preferably from 0.4 cm to 3 cm.

A thickness of the substrate is preferably 500 µm or less, and particularly preferably 300 µm or less.

Examples of a molding method of the microprojections in the present invention include press molding using a mold, injection molding, solution molding, and the like.

The topical drug contains a drug. Examples of drugs usable in the present invention include local anesthetics and antihistamines, as well as anti-inflammatory agents, pain relieving agents, and antipruritic agents.

Examples of the local anesthetic include lidocaine, mepivacaine, dibucaine, bupivacaine, ropivacaine, procaine, chloroprocaine, tetracaine, benzocaine (ethyl aminobenzoate), and the like. Preferred examples of the antihistamine include diphenhydramine and salts thereof, chlorpheniramine and salts thereof, isothipendyl and salts thereof, diphenylpyraline and salts thereof, and diphenylimidazole and salts thereof.

Preferred examples of the anti-inflammatory agents, pain relieving agents, and antipruritic agents include adrenocortical hormone agents such as betamethasone esters, nonsteroidal agents such as diclofenac and indomethacin, skin vitamin agents such as vitamin A, and antibacterial agents such as antibiotics.

As a method of use, a topical drug may be applied to oral mucosa, and thereafter a microprojection patch may be placed thereon so as to cover the topical drug and pressed from a back surface of the substrate. Alternatively, the topical drug may be directly applied to the microprojection surface, applied to oral mucosa, and further backed with an intraoral adhesive tape.

### [Examples]

Hereinafter, the present invention will be described in more detail with reference to the following Examples. These Examples are merely illustrative of the present invention, and the scope of the present invention is not limited thereto.

### Example 1

The microprojection patch shown in Fig. 1 includes a plurality of microprojections standing on a circular substrate. Each microprojection has a length of 300 µm, a truncated conical shape, a tip diameter of 35 µm, and a projection interval of 500 µm. The substrate has a thickness of 60 µm.

For production, a polypropylene sheet having a thickness of 100 µm was placed on a metal concave mold and heated to 180°C, and a microprojection-standing sheet was molded by press molding. The molded sheet was cut into a diameter of 10 mm to produce a microprojection patch. The obtained microprojection patch was backed with an adhesive tape cut into a diameter of 16 mm to obtain an adhesive-tape-backed microprojection patch. The adhesive of the adhesive tape was a mixture of 100 g of an acrylic adhesive (HiPAS10 adhesive, manufactured by CosMED Pharmaceutical) and 30 g of isopropyl myristate, and the base material of the adhesive tape was a polyethylene film having a thickness of 100 µm.

Next, 0.1 g of a topical local anesthetic "Hurricane Gel" (topical drug) (containing 200 mg of ethyl aminobenzoate per 1 g of gel ointment) was spread and placed over an entire surface of the microprojections. In this state, the microprojection patch carrying the local anesthetic was applied to the maxillary region of three volunteers, and the substrate (back surface of the microprojections) was pressed so that the ointment and the microprojection patch were brought into close contact with mucosa using an adhesive bandage tape. After application, at 1 minute, 3 minutes, and 5 minutes, the tape was partially peeled off, and the drug-applied site was lightly pressed with a toothpick to self-evaluate the presence or absence of pain. In all three volunteers, the time until pain was no longer felt was 3 minutes.

For comparison, at the same time, 0.1 g of a topical local anesthetic "Hurricane Gel" (topical drug), containing 200 mg of ethyl aminobenzoate per 1 g of gel ointment (without using the microprojection patch), was applied to a circular area having a diameter of 1 cm on the maxillary region, and the time required until pain was no longer felt was evaluated at 1 minute, 3 minutes, and 5 minutes. As a result, all three volunteers still felt pain after 5 minutes.

### Comparative Example 1

An aqueous solution of hyaluronic acid was cast onto a metal concave mold and dried to form a microprojection-standing sheet, which was then cut into a diameter of 10 mm to produce a microprojection patch. The obtained microprojection patch had a projection length of 300 µm, a projection tip diameter of 35 to 40 µm, a projection interval of 500 µm, and a substrate thickness of 80 µm. The obtained microprojection patch was then backed with an adhesive tape in the same manner as in Example 1 to obtain an adhesive-tape-backed microprojection patch. Subsequent procedures were carried out in the same manner as in Example 1.

When Hurricane Gel was placed on the microprojections, a phenomenon in which the microprojections at the contact portion dissolved was observed. Therefore, Hurricane Gel was first applied to the mucosa, and thereafter the microprojection patch was applied and evaluated (three volunteers). The time until pain was no longer felt was evaluated, and as a result, all three volunteers still felt pain after 5 minutes.

### Example 2

Polylactic acid was dissolved in dichloromethane at a concentration of 10 mass%, cast onto a silicone mold (having truncated conical holes with a depth of 0.3 mm, a tip diameter of 0.04 mm, a base diameter of 0.18 mm, and a hole interval of 0.5 mm), and dried at 60°C to obtain a microneedle sheet. The substrate thickness was 0.1 mm. The sheet was cut into a diameter of 10 mm to obtain a microprojection patch (microneedle patch), which was used while being backed with the same adhesive tape as in Example 1.

Next, 0.15 g of a dental local anesthetic "Neo-Zarocaine Paste" (topical drug), containing 250 mg of ethyl aminobenzoate and 50 mg of diethylaminoethyl p-aminobenzoate hydrochloride per 1 g of paste, was spread and placed over an entire surface of the microprojections. In this state, the microprojection patch carrying the local anesthetic was applied to the maxillary region of three volunteers, and the substrate (back surface of the microprojections) was pressed so that the ointment and the microprojection patch were pressed and brought into close contact with mucosa using an adhesive tape. After application, at 1 minute, 3 minutes, and 5 minutes, the tape was partially peeled off, and the drug-applied site was lightly pressed with a toothpick to self-evaluate the presence or absence of pain. In all three volunteers, the time until pain was no longer felt was 3 minutes.

For comparison, at the same time, 0.15 g of Neo-Zarocaine Paste was applied to a circular area having a diameter of 1 cm in the oral cavity, and the time until pain was no longer felt was evaluated at 5-minute intervals. As a result, all three volunteers still felt pain after 5 minutes.

### Examples 3 to 5 and Comparative Examples 2 to 3

Microprojections shown in Table 1 were produced, backed with the same adhesive tape as in Example 1, and evaluated in the same manner as in Example 2 for actual use. In all cases, the microprojections had a projection tip diameter of 0.04 mm, a projection base diameter of 0.15 mm, and a projection interval of 0.6 mm.

**[Table 1]**

| Example / Comparative Example | Microprojection material | Microprojection length (mm) | Substrate thickness (mm) | Shape | Time to disappearance of pain (min) | Pain upon application and bleeding condition |
|---|---|---|---|---|---|---|
| Example 3 | Polypropylene | 0.25 | 0.1 | Conide | 5 min | No pain; No bleeding |
| Example 4 | Polypropylene | 0.8 | 0.2 | Conical frustum | 3 min | Slight pain; No bleeding |
| Example 5 | Polyglycolic acid | 0.9 | 0.1 | Conical frustum | 1 min | Pain; Bleeding |
| Comparative Example 2 | Polyglycolic acid | 0.9 | 2.0 | Conical frustum | 10 min or longer (the *substrate was thick and did not follow the uneven surface of the maxillary region, resulting in a reduced effect)* | Pain; No bleeding |
| Comparative Example 3 | Polyethylene terephthalate | 0.03 | 0.075 | Conical frustum | 10 min or longer (the *ointment flowed out of the patch when pressing the microprojection patch)* | No pain; No bleeding |

### Examples 6 to 7 and Comparative Example 4

### Application Method and Evaluation Method

A gel prepared by adding polyacrylic acid powder to a spray-type surface anesthetic "Xylocaine Pump Spray 8%" (an aqueous spray containing 8% lidocaine) was used as an application mixture, wherein 1 g of the gel contained 79 mg of lidocaine. An amount of 0.05 g of this gel was used as the application mixture.

The application mixture was spread uniformly over the entire surface of microprojections (diameter: 10 mm) produced from a polypropylene sheet by hot pressing in the same manner as in Example 1. The rear surface of the microprojection patch was backed with an adhesive tape having a diameter of 16 mm. The adhesive tape had the same adhesive composition as that used in Example 1, except that 40 g of polyacrylic acid was added as a hydrophilic substance.

In this state, the microprojection patch was applied to the maxillary region of three volunteers by pressing the substrate (rear surface of the microprojections), and the ointment and the microprojection patch were adhered to the mucosa using the adhesive tape. Two minutes after application, the tape was partially peeled off, and the drug application site was lightly pressed with a toothpick to evaluate the presence or absence of pain by self-assessment.

The viscosity of the application mixture was measured at 25°C using an E-type viscometer (TVE-25, manufactured by Toki Sangyo Co., Ltd.). The sample volume was 0.2 mL.

The results are shown in Table 2.

**[Table 2]**

| Example / Comparative Example | Details of microprojection and coating mixture | Application site | Result after 2 minutes of application | Comment |
|---|---|---|---|---|
| Example 6 | Microprojection: same as Example 1 | Maxillary region | None of the three volunteers felt pain | - |
| | Coating mixture: same as Example 1 | | | |
| | Viscosity: 15,000 mPa·s | | | |
| Example 7 | Microprojection: same as Example 1 | Same as above | One volunteer did not feel pain; two volunteers felt pain | Drug flowed over the skin surface |
| | Coating mixture: not used | | | |
| | Viscosity: 15 mPa·s | | | |
| Comparative Example 4 | Microprojection: same as Example 1 | Upper arm skin | All three volunteers felt pain | Insufficient permeation into upper arm skin |
| | Coating mixture: same as Example 1 | | | |
| | Viscosity: 15,000 mPa·s | | | |

### Examples 8 to 9 and Comparative Example 5

### (Test on Adhesive Strength of Peripheral Adhesive Tape under Wet Conditions)

### Samples

(Sample 1) The adhesive tape used in Example 1
(Sample 2) The adhesive tape used in Example 6
(Sample 3) A rubber-based kraft tape

Each sample was cut into a size of 10 × 40 mm.

### Test Method

Water was sprayed onto the entire surface of high-quality white paper fixed vertically so as to achieve a water amount of approximately 8 mg/cm², thereby wetting the surface. A 10 × 30 mm portion of each sample was adhered vertically to the lowermost end of the white paper and lightly pressed.

After 5 minutes, weights of 10 g and 20 g were applied to the lower end of each sample, and the peeling behavior and falling behavior of the samples from the white paper were observed.

The results are shown in Table 3.

**[Table 3]**

| Example / Comparative Example | Sample | Behavior under load (peeling/falling from white paper) | |
|---|---|---|---|
| | | 10 g load applied | 20 g load applied |
| Example 8 | Adhesive tape used in Example 1 | After 10 minutes under load, remained adhered without peeling | Within 10 minutes under load, peeled and fell |
| Example 9 | Adhesive tape used in Example 6 | Same as above | After 10 minutes under load, remained adhered without peeling |
| Comparative Example 5 | Rubber-based kraft tape | Peeled within 1 minute after load application | Peeled within 1 minute after load application |

The tested HiPAS-based adhesive tapes exhibited extremely high hydrophilicity and, therefore, showed excellent adhesion to wet surfaces. In contrast, the rubber-based adhesive tape was nonpolar, and it was clearly confirmed that its adhesive strength decreased sharply upon contact with water.

In Example 9, it was found that, by adding a hydrophilic resin to the HiPAS adhesive tape, the adhesion performance of the adhesive tape under wet conditions was further improved.

### [Description of Reference Numerals]

- 1: Microprojection patch
- 2: Adhesive tape

## Claims

1. A microprojection patch comprising a substrate and microprojections standing on the substrate,
wherein the microprojection patch is made of a water-insoluble thermoplastic polymer material and is for application to oral mucosa.

2. The microprojection patch according to claim 1,
wherein the microprojections have a tip diameter of 10 µm to 300 µm, a length of 50 µm to 1500 µm, and the substrate has a thickness of 20 µm to 1000 µm.

3. The microprojection patch according to claim 1 or 2,
wherein the density of the microprojections is 10 projections/cm² to 1000 projections/cm².

4. The microprojection patch according to any one of claims 1 to 3, wherein the water-insoluble thermoplastic polymer material is selected from the group consisting of polyolefins, soft silicone, and polyurethane.

5. The microprojection patch according to any one of claims 1 to 3, wherein the water-insoluble thermoplastic polymer material is selected from the group consisting of polyvinyl chloride, polyester, polyacetal, polylactic acid, a lactic acid-glycolic acid copolymer (PLGA), and polyglycolic acid.

6. The microprojection patch according to any one of claims 1 to 5, wherein the substrate has flexibility, and the water-insoluble thermoplastic polymer material has an elastic modulus of 5.0 × 10⁴ kgf/cm² or less.

7. An adhesive-tape-backed microprojection patch, comprising the microprojection patch according to any one of claims 1 to 6 and an adhesive tape,
wherein the microprojection patch is backed with the adhesive tape.

8. The microprojection patch with an adhesive tape according to claim 7,
wherein an adhesive constituting the adhesive tape contains a plasticizer.

9. The microprojection patch with an adhesive tape according to claim 7 or 8,
wherein an adhesive constituting the adhesive tape contains a hydrophilic substance.

10. The microprojection patch with an adhesive tape according to claim 9,
wherein the hydrophilic substance is at least one selected from the group consisting of polyacrylic acid, hyaluronic acid and salts thereof, carboxymethyl cellulose and salts thereof, polyvinyl alcohol, polyvinylpyrrolidone, dextrin, sodium chondroitin sulfate, collagen, and crosslinked products thereof.

11. The microprojection patch with an adhesive tape according to any one of claims 7 to 10,
wherein an adhesive base material constituting the adhesive tape is a foam.

12. The microprojection patch with an adhesive tape according to any one of claims 7 to 10,
wherein an adhesive base material constituting the adhesive tape is a nonwoven fabric.

13. A localized application system for shortening an onset time of a drug on oral mucosa, comprising:
the microprojection patch according to any one of claims 1 to 6; and
a topical drug.

14. A localized application system for shortening an onset time of a drug on oral mucosa, comprising:
the microprojection patch with an adhesive tape according to any one of claims 7 to 12; and
a topical drug.

15. A localized application system for shortening an onset time of a drug on oral mucosa, comprising:
the microprojection patch according to any one of claims 1 to 6; and
a coating mixture.

16. A localized application system for shortening an onset time of a drug on oral mucosa, comprising:
the microprojection patch with an adhesive tape according to any one of claims 7 to 12; and
a coating mixture.

17. The localized application system according to claim 15 or 16,
wherein the coating mixture has a viscosity of 15 mPa·s to 300,000 mPa·s.

18. The localized application system according to claim 13 or 14, wherein a drug contained in the topical drug is a local anesthetic or an antihistamine, and the system is intended for rapid achievement of pain relief or itch relief.

19. The localized application system according to claim 16 or 17, wherein a drug contained in the coating mixture is a local anesthetic or an antihistamine, and the system is intended for rapid achievement of pain relief or itch relief.

20. The localized application system according to claim 18 or 19, wherein the local anesthetic is lidocaine or ethyl aminobenzoate.

21. The localized application system according to claim 18 or 19, wherein the antihistamine is selected from the group consisting of diphenhydramine and salts thereof, and chlorpheniramine and salts thereof.
